# EUROPEAN PATENT APPLICATION

(11) **EP 4 563 121 A1**
(43) Date of publication of application: **04.06.2025**
(21) Application number: 23386127.7
(22) Date of filing: 29.11.2023
(51) Int. Cl.: A61F 2/16

(54) **A CLAMPING DEVICE FOR AN IOL AND AN INTERCHANGEABLE IOL INSERT**

(71) Applicant: Special Account for Research Funds of University of Crete (SARF UoC), 74150 Rethimnon (GR)
(72) Inventor: Tsilimparis, Miltiadis, 70013 Heraklion, Crete (GR); Stavgiannoudakis, Ioannis, 70013 Heraklion, Crete (GR)

(57) **Abstract**

Apparatuses, systems and methods for marking, piercing and suturing an intraocular lens on hand, out of the eye, are described. For example, an intraocular lens, handheld clamping device may include an interchangeable insert. The interchangeable insert may define an oriented and self-guided area, so as to cause the haptics of any specific intraocular lens (IOL) being aligned at specified orientation in such a way to allow the IOL to be marked, pierced or sutured, prior to inserted in an eye.

## Description

### TECHNICAL FIELD

The present disclosure relates to systems, apparatus and methods for extraocular handling of intraocular lenses. Particularly, the present disclosure relates to system apparatus and methods for IOL handheld clamping device, including features for orient, fixing, marking, piercing and suturing of an intraocular lens, for improved sclera support, in absence of capsular one, at cataract surgery operations.

### BACKGROUND

The insertion of an intraocular lens in the absence of capsular support is a special case of cataract surgery with special technical requirements for its performance. Existing options include insertion of the intraocular lens in the anterior chamber, suturing the intraocular lens to the iris, suturing the intraocular lens to the sclera or finally supporting the intraocular lens in the sclera without suturing as described by Tsatsos, M., Vartsakis, G., Athanasiadis, I. et al. "Intraocular lens implantation in the absence of capsular support: scleral fixation" Eye 36, 1721-1723 (2022). https://doi.org/10.1038/s41433-022-02024-3. The last option has several advantages including the closeness of the position of the inserted intraocular lens with the one where the intraocular lens is normally located and its technical simplicity.

The approach of supporting the intraocular lens in the sclera without suturing is currently carried out in two main ways a) the use of a special type of intraocular lens with support anchors through which it is attached to the sclera, as described by Ripa M, Angunawela R, Motta L. "SCLERAL FIXATION OF CARLEVALE INTRAOCULAR LENS: A Systematic Review and Meta-Analysis" Retina. 2023 Oct 1;43(10):1750-1762.), and b) the use of common intraocular lenses that are attached to the sclera either with the help of their haptics or with the use of a suture without stitching as described by Veronese C, Maiolo C, Armstrong GW, Primavera L, Torrazza C, Della Mora L, et al. "New surgical approach for sutureless scleral fixation" Eur J Ophthalmol. 2020;30:612-5. The latter approach offers the great advantage of allowing the insertion of any intraocular lens the surgeon has or wishes. In particular, techniques using sutures for the attachment of intraocular lenses practically allow the surgical attachment of a huge selection of intraocular lenses as described by Yamane S, Sato S, Maruyama-Inoue M, Kadonosono K. "Flanged intrascleral intraocular lens fixation with double-needle technique" Ophthalmology. 2017;124:1136-42.

The suture-based intraocular lens suspension procedure involves the insertion of the support sutures in two or more points in the intraocular lens. Most intraocular lenses are mounted with two sutures. This procedure requires precise antidiametric positioning of the sutures to ensure axial symmetry and to avoid tilting of the intraocular lens along the horizontal axis when inserted as described by Kumar DA, Agarwal A, Prakash G, Jacob S, Saravanan Y, Agarwal A. "Evaluation of intraocular lens tilt with anterior segment optical coherence tomography" Am J Ophthalmol. 2011;151:406-12.e2.

The performance of handling the IOL so as to insert the support sutures and marking of the IOL is not standardized and involves handling difficulties as well as failures in the precise selection of suture insertion points.

Therefore there is a need to provide a standardize procedure for handling the IOL prior to implantation and facilitate the accurate marking and selection of the suture passage points.

### SUMMARY

An aim of the present invention is to provide a clamping device for facilitating handling the IOL prior to implantation that overcome the disadvantages of prior art solutions.

According to an aspect of the present invention, a clamping device for an Intraocular Lens (IOL) , the clamping device comprising:
an elongated clamper body comprising
   a rear end portion defining a handle member configured to be gripped by the user when holding the device,
   a front portion defining a pivot housing having two opposing sidewalls, and
   a bridging portion connecting the rear end portion and the front end portion, the bridging portion comprising an IOL receiving region configured to receive an interchangeable IOL receiving insert containing the IOL; and
a clamping member mounted at one end in the pivot housing and configured to move with respect to the elongated clamper body between a closed and an open position, wherein the clamping member comprises a clamping region having a pressing surface configured, when the clamping member is in the closed position, to apply a predetermine pressing force on the IOL in the interchangeable IOL receiving insert so as to restrict movement of the IOL.

According to embodiment of the present invention, at least a portion of the IOL receiving regionhasa profile that is dimensioned to interlock with a corresponding channel formed on a bottom region of the interchangeable IOL insert so as to clip and secure the interchangeable IOL insert to the IOL receiving region.

According to embodiment of the present invention, the front end comprises a fixing element for securing the clamping member in the pivoting housing of the front portion.

According to embodiment of the present invention, the fixing element forms a rotational hinge about which the clamping member moves between the open and closed positions.

According to embodiment of the present invention, the fixing element is configured to control the fixing force applied to the clamping member by the sidewalls of the pivoting housing.

According to embodiment of the present invention, the fixing element comprises a knob having at an external surface a plurality of marking indicating the forced being applied by the rotational movement of the knob.

According to embodiment of the present invention, the pivoting housing comprises a rear edge configured to engage with the clamping member in the open position so as to restrict its movement.

According to embodiment of the present invention, the clamping region has an inwardly formed contoured profile configured to provide a field of view of at least part of the IOL to the operator when the clamping member is in the closed position.

According to embodiment of the present invention, the clamping region is configured to receive an interchangeable insert for marking, piercing or suturing of the IOL at predetermined locations.

According to embodiment of the present invention, the clamping region comprising locking features configured to interact with corresponding locking features provided on sidewalls of the interchangeable insert so as to clip and secure the interchangeable insert on the clamping region.

According to embodiment of the present invention, the handle member defined by the rear-end portion of clamping body comprises friction gripping formed areas for controlling the positioning of the operator's hand on the clamping body.

According to embodiment of the present invention, the handle member defined by the rear-end portion of the clamping body comprises a flat bottom region configure to maintain the clamping device in a predetermined orientation when positioned on a bench surface, or under a microscope for free hand observation of the IOL.

According to embodiment of the present invention, the rear-end portion of the clamping body comprises a resting pocket configured to receive a rear end of the clamping member when in the closed position.

According to a second aspect, an interchangeable IOL receiving insert for use in the clamping device according to embodiments of the first aspect is provided. The interchangeable IOL receiving insert comprising:
an insert body comprising
a top area defining a concentric pocket configured to receive an IOL haptics, the pocket comprising
   a central pocket region configured to receive an apex portion of the IOL,
   a pair of protruding arch shaped guiding features concentrically positioned with respect to the central pocket region and configured to orient the IOL haptics at a specific position, and
   one or more wall regions positioned at an outer edge of the concentric pocket configured to form a protective wall for the IOL; and
a bottom area defining a channel having a profile that is dimensioned to interlock with a corresponding profile of a locking region of the clamping tool body so as to clip and secure the interchangeable IOL insert to the IOL receiving region of the clamping tool body.

According to embodiments of the second aspect of the present invention, the interchangeable IOL receiving insert of claim 14, wherein the concentric pocket further comprises a pair of opposing slots oriented perpendicular to a longitudinal axis of the clamping member, each slot comprising an opening for receiving a suturing needle, wherein the slots are positioned at a predetermine distance from the central pocket region.

### BRIEF DESCRIPTION OF THE DRAWINGS

The following drawings are provided as an example to explain further and describe various aspects of the invention.
FIG. 1 is a perspective view of an example extraocular *IOL* handheld operated clamping device.
FIG. 2 shows a longitudinal cross-sectional view of the extraocular *IOL* handheld operated clamping device..
FIG. 3 is a perspective view of a distal portion of an example clamping device body of the extraocular *IOL* handheld operated clamping device of FIG. 1
FIG. 4 is a perspective view of a distal portion of an example clamping device, clamping member of the extraocular *IOL* handheld operated clamping device of FIG. 1
FIG. 5 is a perspective view of a distal portion of an example clamping device fixing element of the clamping member of the extraocular *IOL* handheld operated clamping device of FIG.1
FIG. 6 is a perspective view of a distal portion of an example clamping device lens receiving insert, of the extraocular IOL handheld operated clamping device of FIG.1
FIG. 7 is a perspective view of an example extraocular *IOL* handheld operated clamping device in open position.
FIG. 8 is a perspective view of an example extraocular *IOL* handheld operated clamping device in fixed position.
FIG. 9 shows an example of an IOL.
FIG. 10 shows an example of a toric IOL.
FIG. 11 shows a side view of an example clamping member of the extraocular *IOL* handheld operated clamping device of FIG.1
FIG.12 shows a perspective view of the piercing insert, locked in position on the clamping member of the extraocular *IOL* handheld operated clamping device of FIG.1
Fig. 13 shows a perspective focused view of the middle portion of an example clamping member of an extraocular *IOL* handheld operated clamping device.
FIG. 14 shows a perspective focused view of the front portion of an example extraocular *IOL* handheld operated clamping device of FIG. 1.
FIG.15 shows a perspective view of an example of an IOL piercing insert of the extraocular IOL handheld operated clamping device of FIG.1
FIG. 16 shows a top side view of an example clamping device, lens receiving insert.
FIG. 17 shows an example suturing needle.
FIG. 18 shows a top field of view from the operator's sight, of the clamping member over the interchangeable IOL receiving insert.
FIG. 19 shows a top side view of an example clamping device, lens receiving insert, with an IOL in position.
FIG. 20 shows a top field of view from the operators sight, of the clamping member over the interchangeable IOL receiving insert, with an IOL in position and a suturing needle in position performing a suture action.
FIG.21 shows a cross sectional view of the pivot hinge of an example clamping device body of the extraocular *IOL* handheld operated clamping device of FIG. 1

### DETAILED DESCRIPTION

For the purposes of promoting an understanding of the principles of the present disclosure, reference will now be made to the implementations illustrated in the drawings, and specific language will be used to describe the same.

It will nevertheless be understood that no limitation of the scope of the disclosure is intended. Any alterations and further modifications to the described devices, methods and any further application of the principles of the present disclosure are fully contemplated as would normally occur to one skilled in the art to which the disclosure relates. In particular, it is fully contemplated that the features, components, and/or steps described with respect to one implementation may be combined with the features, components and/or steps described with respect to other implementations of the present disclosure.

The present disclosure relates to systems, apparatuses and methods for clamping and suturing an IOL, prior to implantation into an eye of a patient.

Particularly, the present disclosure relates to systems, apparatuses and methods for IOL extraocular handheld clampers, having features for precise and accurate suturing of an IOL, prior to insertion.

In general, the present disclosure relates to an extraocular handheld operated clamping device of an intraocular lens, (IOL), that may include an IOL receiving interchangeable insert , a holder body having an IOL receiving region for receiving interchangeable insert, and a clamping member, and fixing element. The clamping device may include a longitudinal axis extending centrally along the holder body , that may include a front portion, a rear portion and a bridge portion that connect the two previous portions. The front portion may include a pivot hinge housing, which is formed between two opposing sidewalls that permit a limited rotational movement of the clamping member in open and closed positions. The pivot housing compartment may include a fixing element that forms the pivot axis and apply a predictable force to the sidewalls of the pivot housing in order to fix in place the clamping member in desirable position. The clamping member may include a lever arm, partly housed in the pivot housing, that may extend upper the central part of the loaded IOL, with a pressing region on it to press firmly the IOL into position without damage, and a top viewed contour that permit unobstructive view of the operating area. The bridge portion may include an interchangeable insert housing compartment, that receiving different types of interchangeable inserts in order to house different types of intraocular lenses ("IOL"), for loading on the clamping device. The interchangeable IOL receiving insert may include specific regions on the top surface, as to housing at predetermined orientation and position an appropriate type of intraocular lens each time, with features that ensure the precise marking, piercing and suturing of the IOL, and also specific regions on the bottom surface in order to be locked in place on the corresponding regions at the bridge portion of the clamping device. The rear portion may include a hand grip surface and anatomically contoured handle part, that serves the precise control and handling of the clamping device by the operator. The interchangeable IOL receiving insert may include a rotational IOL receiving region, in order to allow toric IOL's to oriented radially for precise marking, piercing or suturing. The rear portion of the clamping device body, may include a thumb operated rotation mechanism, for the precise rotation of the toric IOL receiving region of the interchangeable IOL receiving insert prior to be marked, pierced or sutured.

In exemplary embodiments, the fixing element may include an applied force adjusting knob, with friction aided surface, for precise control by the user and marking indicators on it for predictable force load setting. The fixing element may further include an eccentric cam lever clamping mechanism in place of previous screwing bolt system, that permits simpler and more secure application.

In exemplary embodiments, the pivot housing may include a front wall configured to act as a rotational angle limiter of the clamping member rotation, in contact with the corresponding shaped bottom part of the clamping member.

In exemplary embodiments, the clamping member may have a shorter length as to reduce the applied torque on the pivot axis and the pressing point region of it by the operators hand action.

In further exemplary embodiments, the clamping member of the clamping device may include an IOL clamping region, that can receive interchangeable inserts for precise marking or piercing of the IOL. These inserts act as aid devices at the performed operations on the IOL, or as independent operating devices. The piercing interchangeable insert, may include additional feature of combined suturing action, through the use of special configured needles.

It is to be understood that both the foregoing general description and the following detailed description are exemplary and explanatory in nature and are intended to provide an understanding of the present disclosure without limiting the scope of the present disclosure. In that regard, additional aspects futures and advantages of the present disclosure will be apparent to one skilled in the art from the following detailed description.

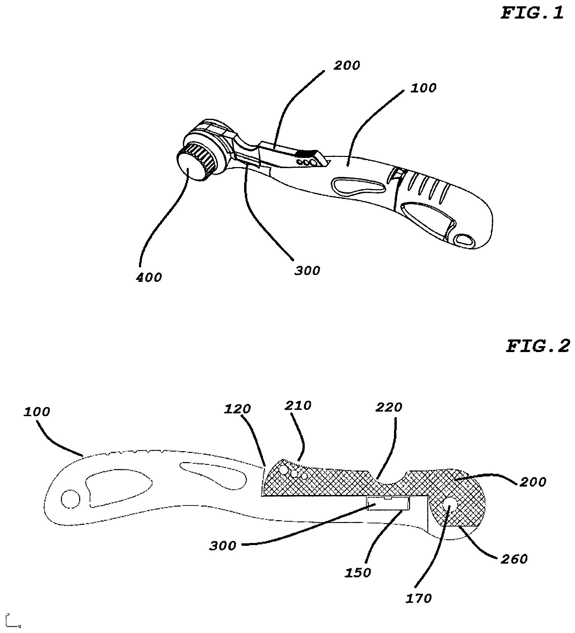

FIG. 1 shows an example extraocular IOL handheld operated clamping device, that includes a clamper body 100, a rotational and adjustable IOL clamping member 200, a screwing rotational fixing element 400, a compartment operable to house an interchangeable IOL receiving insert 150, and an interchangeable IOL receiving insert 300.

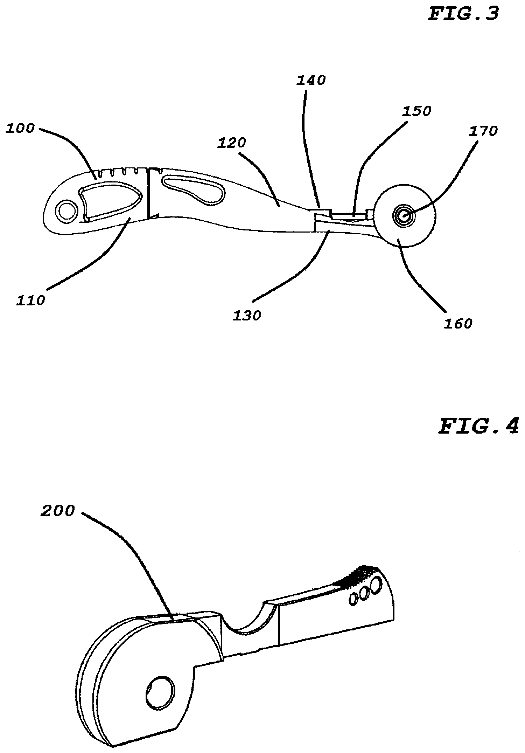

FIG. 2 shows a longitudinal cross-sectional view an example extraocular IOL handheld operated clamping device according to embodiments of the present invention. The positions of the clamping member 200, interchangeable IOL receiving Insert 300 and pivot point 240, as shown in figure 3. The clamping member 200 comprises a clamping region 220 having an IOL pressing surface 230. The clamping region 230 has a thinned silhouette contour, so as to provide a clear field of view of the IOL optical disk to the operator, when the clamping member 200 is in the closed position.

FIG.3 shows a side view of the extraocular IOL handheld operated clamping device body according to embodiments of the present invention. An anatomically shaped handheld rear portion 110, which in some instances has a lightweighting structure with openings distributed in a predetermined pattern on the handle portion in other instances may have a flat bottom shape that permits leveled positioning of the device on a bench, in other instances may have any desired contour. The region 120 of the clamping device body 100, may form a pocket that can be used in a number of different ways depending on the design of the clamping device. For example, the, the region 120 may receive the rear end 210 of the clamping member 200. In a further example, region 120 may form a friction gripping surface, for use with a short lever clamping member 200 designed to reach the rear end of the interchangeable IOL receiving insert. In yet further examples, region 120 may house a mechanism that controls the rotation of the toric IOL receiver, of a specific interchangeable, toric IOL receiving insert. Region 130of the clamping device body 100, forms a bridge, that connects the rear portion 110 of the body 100, to the front portion 160, which works as clamping member pivot housing. Also, the upper portion of the region 130, forms a IOL receiving region 150 with clipping aid shape, that hold and secure in position the interchangeable IOL receiving insert 300. The front portion 160 of the clamping device body 100, forms a clamping member 200, pivot housing. The pivot housing portion of the body has two side walls which enclose the 250 portion of the clamping member 200 and a transverse hole 170 trough which pass the fixing element 420 pivot axis.

FIG.4 shows a perspective view of the clamping member device according to embodiments of the present invention. The specific clamping member is the long one version, in which they showed clearly, the individual parts of it, as the pivot axis hole , the flat bottom area that acts as a stopper in open position, the friction gripping area at the front, for better operator's finger positioning, the IOL pressing point region and the slimline contour of the region over it.

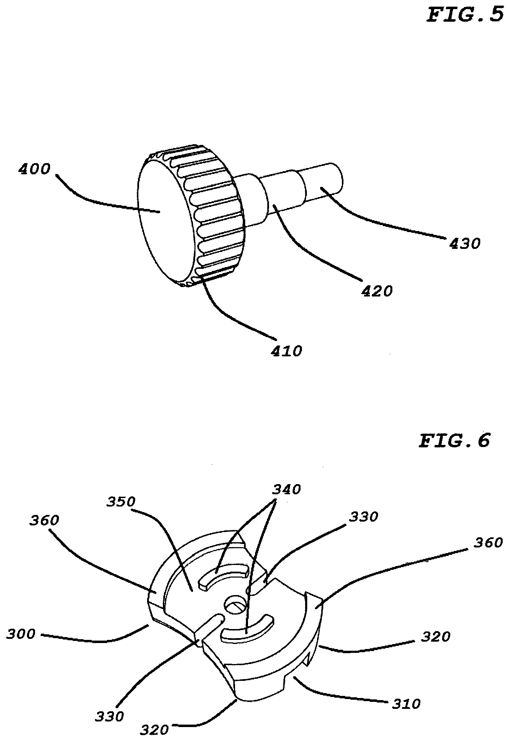

FIG.5 shows a perspective view of a fixing element 400 of the clamping device according to embodiments of the present invention. The fixing element 400 may be provided with a knurled or other friction aided shape for secure handling formed knob end 410, connected with a coaxial cylindrical part which act as a bearing for load transfer to the clamping device body, forms a cylindrical pivot area 420 that permits the clamping member 200, to rotate freely on it and has a threaded portion 430 which in series screwed in clamping device body threaded part 170, so it can adjust the fixing force that keeps in place the clamping member 200. The 410 knob may have force indicator markings on it, to adjust the required force at predefined levels.

FIG.6 shows a perspective view of an extraocular clamping device interchangeable IOL receiving insert 300 according to embodiments of the present invention. The interchangeable IOL receiving insert 300 includes a specific profiled bottom channel 310, that matches the profile of the 150 portion of the clamping device, as to clip end secure in place the interchangeable IOL receiving insert 300, a specific profile shaped bottom 320 that helps the operator to hold the piece in hand and also forms a clipping area that secure in place the interchangeable IOL receiving insert 300, two radially and antidiametrically positioned guide slots 330, through them the operator drive the suturing needle in position, ensuring the radius distance of the hole, as also the angle, in respect of IOL orientation. A pair of protruding arch shaped guiding features 340 in antidiametrically positions in respect of the rotational axis of the IOL, where they form a region that secure the optical diameter of the IOL disk and also due to their position receive and lock the IOL haptics at specific anytime angle. An eccentric to previous region outer one 350, that receiving the haptics far most diameter and limited by the 360 part of the insert, which act as a protection wall to the received IOL. The shape and dimensions of the positioned guiding slots 330 is determined according to the shape and dimensions of the IOL and the IOL haptics.

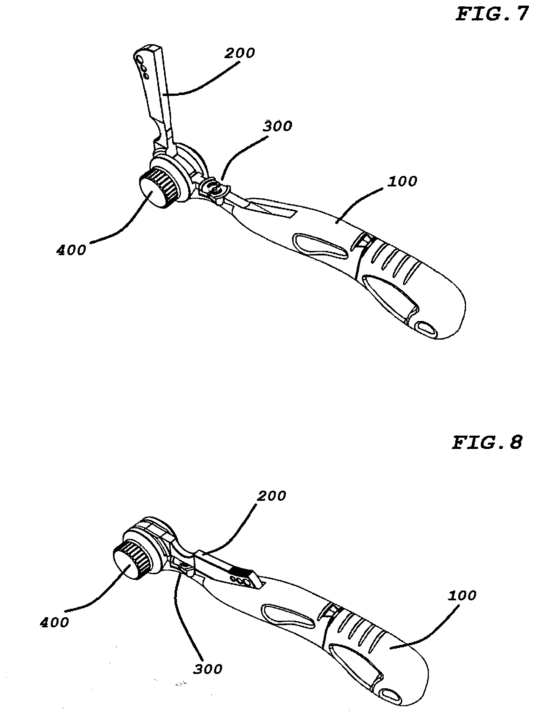

FIG.7 shows a perspective view of the extraocular clamping device with the clamping member 200 in open position according to embodiments of the present invention. The handheld body 100, the interchangeable IOL receiving insert 300, the fixing knob 400 and the clamping member 200 in open position perpendicular to the longitudinal axis of the extraocular IOL clamping device, that permits the operator to place the IOL in the device.

FIG.8 shows a perspective view of the extraocular clamping device with the clamping member 200 in closed position and the relative positions of the major parts of the device according to embodiments of the present invention. The interchangeable IOL receiving insert 300 and its relative position to the press point 230 of the clamping member 200 it is clearly visible.

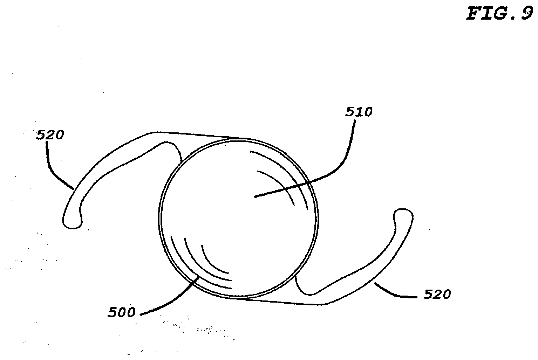

FIG.9 shows an example of an Intraocular Lens (IOL) 500 according to embodiments of the present invention. An IOL includes the central lenticular shaped optical portion 510, that usually has a diameter of six millimeters, the haptics of the lens 520, antidiametrically positioned to the longitudinal axis of the lens, that serve to the fixing of the lens at position in the eye and usually are flat shaped elastic bands or threads.

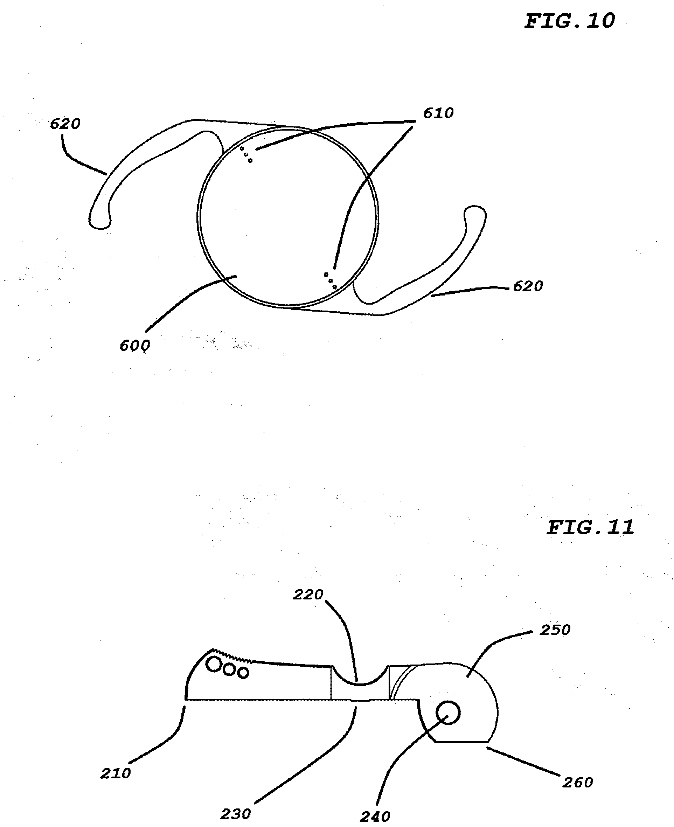

FIG. 10 shows an example of a toric IOL 600 according to embodiments of the present invention. A toric IOL has the same specifications as the IOL example of FIG.9, plus includes specific markings 610 on its periphery, as indicators of the toric axis. 610 markings has to be set on the interchangeable IOL receiving insert IOL receiving region, by specified orientation. This is achieved by the help of rotational IOL ragion mechanism of the interchangeable IOL receiving insert in toric version of it.

Fig. 1 1 shows a side view an example of the clamping member 200 according to embodiments of the present invention. The pivot point 240, the middle portion of the clamping member 220 in the area over the IOL pressing point 230 with a thinned silhouette contour, as to provide clear field of view of the IOL optical region to the operator and also shaped in form to allow a marking or piercing insert, to be locked on it, as to permit precise marking and piercing of an IOL at specified points, the rear portion 210 that serves as an operators thumb griping area, or can be eliminated, as to form a shorter version of the clamping member, with less torque on the rear side of the pressing point 230, the 240 pivot hole that receive the pivot axle of the fixing element 400 and the special profiled region 260, which acts as a rotation angle limiter of the clamping member 200 in open position.

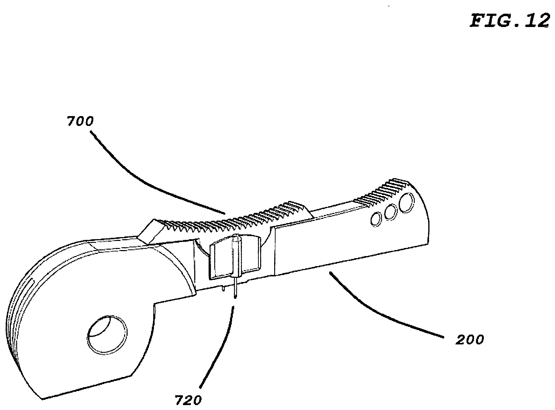

Fig. 12 shows a perspective view of an example of a clamping member 200 of the extraocular IOL clamping device, with an example of a piercing insert tool 700 attached on it, at a position ready to perform an IOL pierce according to embodiments of the present invention. A pair of piercing needles 720, are integrated in the piercing tool insert body at an ideal distance and orientation regard to the IOL, and the whole insert is locked in position by the help of the clipping action specific shaped regions of the two parts.

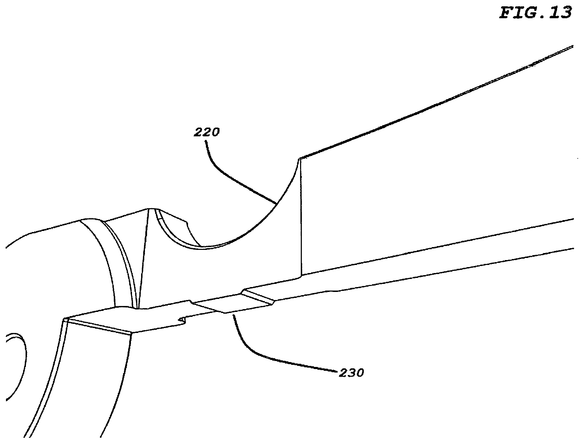

Fig. 13 shows a detailed perspective view of the central portion 220, also referred to as clamping region, of the clamping member 200 of the extraocular IOL clamping device according to embodiments of the present invention. The clamping region 220 of the tool is shaped according to match the shape of the piercing or marking interchangeable inserts, so that it permits the secure clipping of the inserts on it as to perform the specified actions The clamping region 220 comprises a pressing region 230 that is provided with has a specific shape which enables, when the clamping member 200 is in the closed position, to secure the IOL firmly in position, while not stressing the IOL, so as to allow the operator to perform a marking, piercing or suturing operation of an IOL.

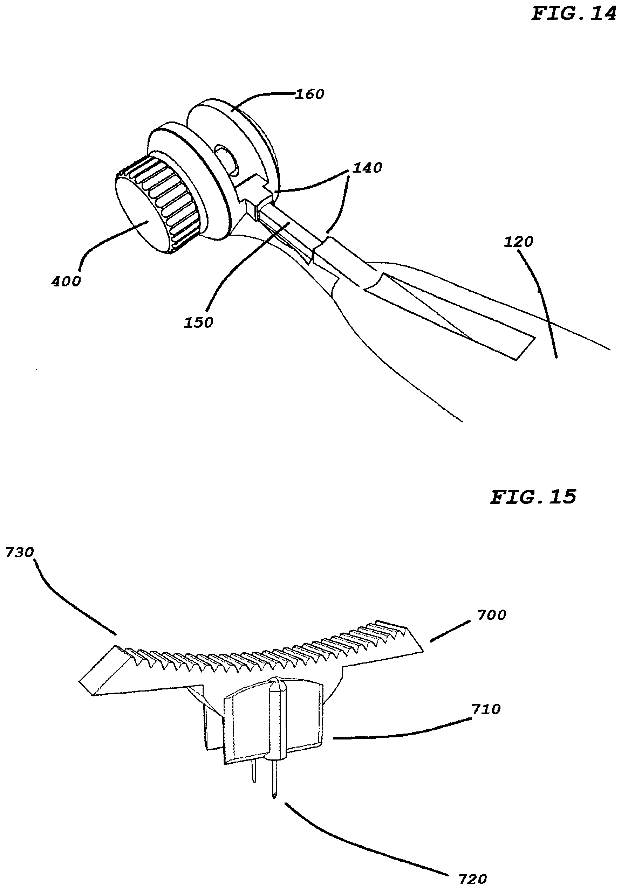

Fig. 14 shows a perspective focused view of the front portion part of extraocular IOL clamping device according to embodiments of the present invention. The clamping member 200 and the interchangeable insert 300 are omitted for clarity. The 150 is the interchangeable insert receiving region, with a shape that matching the shape of the interchangeable insert 300 tunnel and specific clipping aid profile, as to secure the insert in position. The vertical, radius shape formed walls 140, support the insert in place and permit the optional rotational movement in the toric version of it. The 160 front portion of the extraocular IOL clamping device body, support the rotational movement of the clamping member 200 and the side loads on it due the performed handling by the operator. They also include the pivot hole that receive the pivot axis of the fixing element 400 and withstand the force of the fixing bolt to the clamping member 200.

Fig. 15 shows a perspective view of an example of the interchangeable piercing insert 700 according to embodiments of the present invention. The interchangeable piercing insert 700 comprises high friction regions 730, that help the operator to apply with his fingers a downward force as to lock the insert in position and perform the piercing action on the IOL. The side walls 710, act as sliders on the sides of the clamping member 200, that guide the insert in its final position without side movement and tilting. The side walls 710 also include on the inner side, specific profile pins that matching respective slots on the sides of clamping member 200, as to ensure the precise vertical movement of the insert 700 in position. The piercing needles 720 are permanently fixed in the sides of insert 700 at specific distance and orientation regarding the center of the IOL and they have a suitable length to reach the bottom of the IOL and extend beyond it at a safe length.

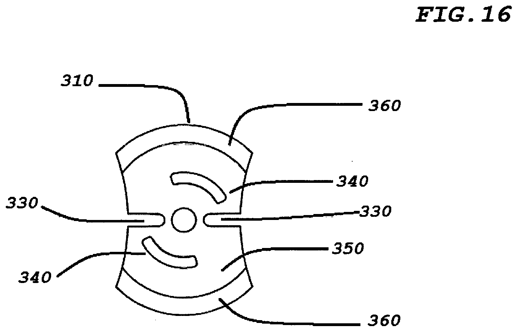

Fig. 16 shows a top view of an example of an interchangeable IOL receiving insert 300 according to embodiments of the present invention. The purpose of this insert is to accommodate an IOL prior to perform a marking, piercing or suturing action on it. Perpendicular to the longitudinal axis two antidiametrically positioned guide slots 330, have enough length and wide dimensions as to permit the hand held suturing needle, or alternative, the piercing tool needles, to pass through and reach the opposite side of the IOL without obstruction problems and also ensuring the right radius distance of the hole and the angle, in respect of lens orientation. The protruding arch shaped guiding features 340 in antidiametrically positions in respect of the rotational axis of the IOL, forms an internal pocket that secure the optical diameter of the IOL disk and also due to their position receive and lock the IOL haptics at specific anytime angle. An eccentric to previous pocket outer one 350, accommodate the haptics of an IOL and the 360 part of the insert act as a protection wall to the accommodated IOL.

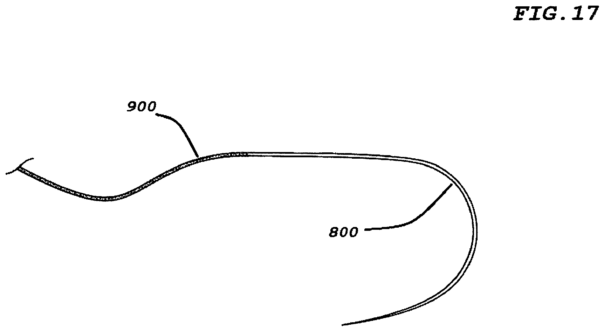

Fig. 17 shows an example of a suturing needle 800 that comprising a circle shaped metal tip and a flexible but strong long thread part 900 connected to the tip according to embodiments of the present invention. The operator has to insert the needle through the slots 330 of the interchangeable IOL receiving insert, prior to penetrate the optical disk of the IOL at the optimal radius that specified by the end of the slot 330.

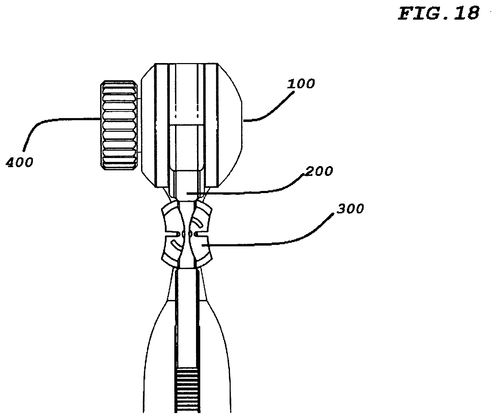

FIG. 18 shows a detailed top view of the front end of the extraocular IOL clamping device that also represents the operators eye view over the IOL position on the device. The clamping region at the center of the clamping member 200 has a slimline shape defined by the inward contoured profile compared to the other regions of the clamping member 200. The profile of the clamping region permits unobstructed view of a predetermined portion of the IOL to the operator so as to perform the suturing or marking of the IOL. As shown in figure 18, when the clamping member 200 is in the closed position, the profile of the clamping region enables the operator to view certain feature so the interchangeable IOL such as the receiving insert 300 with the suturing guide slots 330, the IOL haptic fixers, the protruding arch shaped guiding features 340, the fixing element 400, the front portion of the extraocular IOL clamping device body 100 that forms the pivot hinge of the rotational clamping member 200.

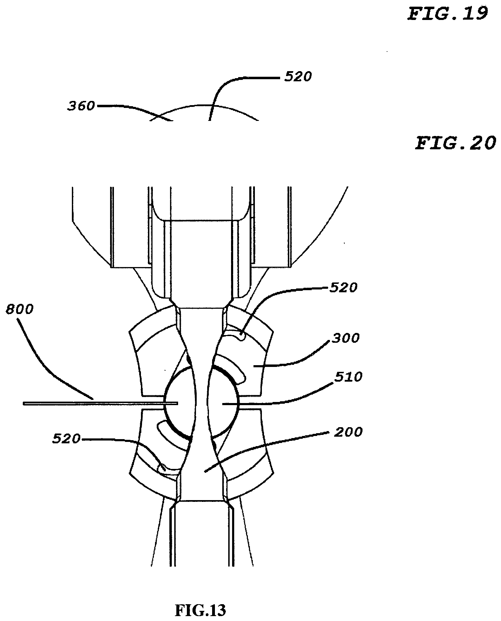

Fig. 19 shows a top view of an example of interchangeable IOL receiving insert 300, with an IOL fitted in position, and the corresponding relative orientation of both the IOL and the receiving insert 300 according to embodiments of the present invention. the protruding arch shaped guiding features 340 and the outer side walls 360, set the radius limits respectively, of the central optic disk of the IOL 510, and the outer ends of the IOL haptics 520. The position of 340, adapted to the occasional IOL shape and at fixed version are fixed placed in the pocket 350, while at rotational -toric version follow the rotated IOL receiving plate.

Fig.20 shows a top view of the clamping device with the clamping member 200 in the closed position over the interchangeable IOL receiving insert 300 containing an IOL in position and a suturing needle 800 in position performing a suture action. The contoured profile of the clamping member 200 over the IOL, provides the operator with a clear view of the IOL operational area allowing for the unrestricted marking, piercing, or suturing of the IOL. Full protection of the sensitive parts of the IOL, like the haptics 520 and the optical disk area 510, is provided by the formed pockets and diaphragms on the insert, which is also clearly visible in figure 20. The clamping device of the present invention enables the operator to handle the IOL with unrestricted movements by one hand, in three axes, during marking, suturing or piercing of the IOL.

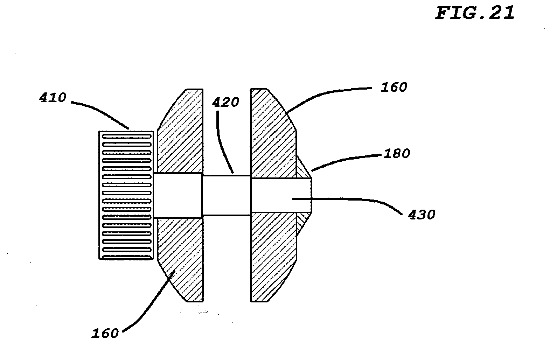

Fig.21 shows a top cross-sectional view of the pivot hinge of an example front portion of the extraocular IOL handheld operated clamping device according to embodiments of the present invention. The front portion of the clamping body 100 comprise two supporting wall 160, which define the pivoting housing. The front-end of the clamping member 200 is secured on the pivoting axis 420 which allows for clamping member 200 to pivot between the open and closed positions. A knurling formed fixing knob 410 may be provided on one end of the pivoting axis 420, on the outside of one of the supporting walls 160, while the other end of the pivoting axis 420 comprises a male threaded region which is configured to interact with a female threaded opening on the other sidewall 160, when the fixing knob 410 is rotated. In general, the pivoting axis 420 is arranged in the form of a screw, which configured by means of the rotational motion of the knob 410 to adjust the fixing torque applied to the clamping member 200 by the opposing sidewalls of the front portion160.

In general, the present clamping device may be used in a sterile surgical field, where the surgeon takes the clamping device, loosens the fixing knob 400 and set the clamping member 200, at open position. After selecting the appropriate interchangeable IOL receiving insert 300 for the available intraocular lens, the insert is placed in the tool holder; the intraocular lens is placed in the insert in the orientation resulting from the embossing of the insert. If required, the insert shall be rotated to obtain the desired position of the astigmatic axis. After confirming the correct position of the intraocular lens in relation to the passage slots 330 of the support sutures, the surgeon lowers the clamping member to trap the intraocular lens in the desired position and rotates the fixing knob until clamping member and insert-intraocular lens assembly is immobilised. The correct position of the suture passage slots shall be checked again. Next, while the intraocular lens is held in position with one hand, the surgeon passes the support sutures successively through the points resulting from the position of the insertion notches. After the sutures have been passed through, their ends are cauterised to produce a small knot to prevent their passage through the suture passage hole. The intraocular lens is then released from the tool, removed from the tool and ready for insertion and surgical fixation.

### Reference numeral

- 100: clamping device body
- 110: rear portion of the body
- 120: front end of rear portion of the body
- 130: bridge portion of the body
- 140: radius shape formed walls
- 150: interchangeable insert receiving region
- 160: front portion of the body
- 170: transverse hole of pivot axis
- 200: clamping member
- 210: rear portion of the clamping member
- 220: middle portion of the clamping member
- 230: pressing point - clamping region
- 240: pivot axis hole of the clamping member
- 250: front portion of the clamping member
- 260: rotation angle limiter region
- 300: interchangeable IOL receiving Insert
- 310: bottom channel
- 320: special profiled bottom
- 330: needle guide slots
- 340: protruding arch shaped guiding features
- 350: haptics accommodation region
- 360: outer diameter side walls
- 400: fixing element
- 410: fixing knob
- 420: pivot axis
- 430: male threaded portion
- 500: Intraocular Lens (IOL)
- 510: optical region of an IOL
- 520: haptics of an IOL
- 600: toric IOL
- 700: piercing insert tool
- 710: side walls of a piercing insert tool
- 720: piercing needles of a piercing insert tool
- 730: high friction regions of a piercing insert tool
- 800: suturing needle
- 900: suturing thread

## Claims

1. A clamping device for an *Intraocular Lens, IOL,* the clamping device comprising:
an elongated clamper body (100) comprising
a rear end portion (110) defining a handle member configured to be gripped by the user when holding the device,
a front portion defining a pivot housing having two opposing sidewalls (160) , and
a bridging portion (130) connecting the rear end portion (110) and the front end portion, the bridging portion (130) comprising an IOL receiving region (150) configured to receive an interchangeable IOL receiving insert (300) containing the IOL; and
a clamping member (200) mounted at one end in the pivot housing and configured to move with respect to the elongated clamper body (100) between a closed and an open position, wherein the clamping member (200) comprises a clamping region ( 220) having a pressing surface (230) configured, when the clamping member (200) is in the closed position, to apply a predetermine pressing force on the IOL in the interchangeable IOL receiving insert (300) so as to restrict movement of the IOL.

2. The clamping device of claim 1, wherein at least a portion of the IOL receiving region (150) hash a profile that is dimensioned to interlock with a corresponding channel (310) formed on a bottom region of the interchangeable IOL insert (300) so as to clip and secure the interchangeable IOL insert (300) to the IOL receiving region (150).

3. The clamping device of any one of claims 1 or 2, wherein the front end comprises a. fixing element (400) for securing the clamping member (200) in the pivoting housing of the front portion.

4. The clamping device of claim 3, wherein the fixing element (400) forms a rotational hinge about which the clamping member (200) moves between the open and closed positions.

5. The clamping device of claim 3 or 4, wherein the fixing element (400) is configured to control the fixing force applied to the clamping member (200) by the sidewalls of the pivoting housing.

6. The clamping device of claim 5, wherein the fixing element (400) comprises a knob (410) having at an external surface a plurality of marking indicating the forced being applied by the rotational movement of the knob (410).

7. The clamping device of any one of the preceding claims, wherein the pivoting housing comprises a rear edge configured to engage with the clamping member (200) in the open position so as to restrict its movement.

8. The clamping device of any of the preceding claims, wherein the clamping region (220) has an inwardly formed contoured profile configured to provide a field of view of at least part of the IOL to the operator when the clamping member (200) is in the closed position.

9. The clamping device of claim 8, wherein the clamping region (220) is configured to receive an interchangeable insert (700) for marking, piercing or suturing of the IOL at predetermined locations.

10. The clamping device of claim 9, wherein the clamping region (220) has a profile that comprising locking features configured to interact with corresponding locking features provided on sidewalls (710) of the interchangeable insert (700) so as to clip and secure the interchangeable insert (700) on the clamping region (220).

11. The clamping device of any one of the preceding claims, wherein the handle member defined by the rear-end portion (110) of clamping body (100) comprises friction gripping formed areas for controlling the positioning of the operator's hand on the clamping body (100).

12. The clamping device of any one of the preceding claims, wherein the handle member defined by the rear-end portion (110) of the clamping body (220) comprises a flat bottom region configure to maintain the clamping device in a predetermined orientation when positioned on a bench surface, or under a microscope for free hand observation of the IOL.

13. The clamping device of any one of the preceding claims, wherein the rear-end portion (110) of the clamping body comprises a resting pocket (120) configured to receive a rear end of the clamping member (200) when in the closed position.

14. An interchangeable IOL receiving insert (300) for use in the clamping device of claims 1 to 13, the interchangeable IOL receiving insert (300) comprising:
an insert body comprising
a top area defining a concentric pocket (350)configured to receive an IOL haptics, the pocket comprising
a central pocket region configured to receive an apex portion of the IOL,
a pair of protruding arch shaped guiding features (340) concentrically positioned with respect to the central pocket region and configured to orient the IOL haptics at a specific position, and
one or more wall regions (360) positioned at an outer edge of the concentric pocket configured to form a protective wall for the IOL; and
a bottom area defining a channel (310) having a profile that is dimensioned to interlock with a corresponding profile of a locking region of the clamping tool body so as to clip and secure the interchangeable IOL insert (300) to the IOL receiving region (150) of the clamping body (100).

15. The interchangeable IOL receiving insert (300) of claim 14, wherein the concentric pocket (350) further comprises a pair of opposing slots (330) oriented perpendicular to a longitudinal axis of the clamping member (200), each slot (330) comprising an opening for receiving a suturing needle, wherein the slots are positioned at a predetermine distance from the central pocket region.
